Europäisches Patentamt

European Patent Office

Office européen des brevets

(19)

(11) Publication number: **0 570 606 A1**

# EUROPEAN PATENT APPLICATION

(12)

(21) Application number: **92108360.6**

(22) Date of filing: **18.05.92**

(51) Int. Cl.5: **A61K 9/50**

(43) Date of publication of application:
**24.11.93 Bulletin 93/47**

(84) Designated Contracting States:
**DE DK ES FR GB IT NL SE**

(71) Applicant: **Wakamoto Pharmaceutical Co., Ltd.**
**5-3, Nihonbashimuro-machi 1-chome**
**Chuo-ku, Tokyo(JP)**

(72) Inventor: **Ogasawara, Yoshiaki**
**101-1-3, Hina**
**Fuji-shi, Shizuoka-ken(JP)**
Inventor: **Kageyama, Shinji**
**280, Kanate,**
**Ohimachi**
**Ashigara-Kani-gun, Kanagawa-ken(JP)**
Inventor: **Oguma, Toru**
**450-62, Ojiri**
**Hadano-shi, Kanagawa-ken(JP)**

(74) Representative: **VOSSIUS & PARTNER**
**Postfach 86 07 67**
**D-81634 München (DE)**

(54) **Orally administrable solid medicine and process for preparing the same.**

(57) The present invention relates to an orally administrable solid medicine and a process for preparing the same. The medicine comprises a medicinal component or a solid medicinal component having an uncomfortable taste and a polymer film coating covering said medicinal component or a solid medicinal component. The polymer film coating comprises a film forming polymer and an adhesion/aggregation preventing agent selected from the group consisting of starch particles having an average particle diameter of 15 $\mu$m or smaller and fine crystalline cellulose particles having an average particle diameter of 25 $\mu$m or smaller. According to the present invention, the medicine particles are free of adhesion or aggregation, have a good absorption ability of the medicinal component, and are easily produced without using special apparatuses and techniques.

EP 0 570 606 A1

EP 0 570 606 A1

The present invention relates to an orally administrable solid medicine wherein the uncomfortable taste (e.g., bitter taste and acid taste) of a medicinal component is hidden or masked, and in particular, to an orally administrable solid medicine wherein the uncomfortable taste of a medicinal component is hidden or masked; even infants can safely take the medicine; and the medicine has a high bioavailability.

As generally accepted processes for masking an uncomfortable taste of a medicinal component contained in an orally administrable solid medicine, there have been established processes for coating the medicinal component with films of sugar, water-soluble cellulose ethers and intestinal juice soluble polymers. It is considered that these processes are most effective, in particular, for the preparation of tablets.

However, granules, powder and pellets have very large surface areas. In order to provide sufficient masking effects to these medicines, the coating amount must be increased, which leads to the problem that the particles of the medicines easily adhere or aggregate to each other due to the tackiness of a film forming polymer. As a result, the particles inevitably become large and cause a coarse feel, and therefore it becomes difficult to swallow the medicine. Accordingly, the resultant medicine is not suitable for infants.

It is known that inorganic materials such as talc, titanium oxide and silicon dioxide are added to the film forming polymer in order to reduce or eliminate its tackiness. However, such inorganic materials cannot sufficiently work to eliminate such tackiness.

Under these circumstances, as processes for masking the uncomfortable taste of the medicinal component contained in medicines in the form of granules, powder and pellets, there have recently been proposed several processes, such as a microcapsulation process with ethyl cellulose using phospholipid as a phase separation inducing agent (JP-B-60-45 845);

a process for causing a medicinal component to collide with titanium oxide, talc and wax, whereby forming a coating on it (JP-A-63-301 815); and

process for dropping a suspension of a medicinal component and a polymer binder into a powder bed of gastric juice soluble polymer particles, to form a coating (JP-A-63-277 616).

However, there are drawbacks, for example, that these processes necessitate special apparatuses, operations or techniques and that the use of ethyl cellulose or wax prevents the absorption of the medicinal component in the body, although the uncomfortable taste can be masked.

One object of the present invention is to provide an orally administrable solid medicine having a relatively small size, such as granules, powder or pellets, wherein a medicinal component or a solid medicinal component is coated with a film forming polymer, the medicine being free of adhesion or aggregation therebetween and having a good absorption ability of the medicinal component, and being easily prepared without any special apparatuses or operations.

Another object of the present invention is to provide a process for manufacturing such a medicine.

The present inventors have made intensive studies on various additives which seem to prevent adhesion and/or aggregation of the medicine particles in film coating processes for medicinal components, granules, powder or pellets including a medicinal component in conventional apparatuses and, as a result, have found that certain types of starch or cellulose have a good preventing effect for the adhesion/aggregation of the medicine particles. The present invention has been made on the basis of this finding.

The present invention relates to an orally administrable solid medicine comprising a medicinal component or a solid medicinal component having an uncomfortable taste and a polymer coating covering the medicinal component or a solid medicinal component, wherein the polymer film coating comprises, based on the total weight of the orally administrable solid medicine, the following components:

(1) 5-40 wt.% of a film forming polymer, and

(2) 2-60 wt.% of an adhesion/aggregation preventing agent selected from the group consisting of starch particles having an average particle size of 15 $\mu$m or smaller and fine crystalline cellulose particles having an average particle size of 25 $\mu$m or smaller.

The present invention also relates to a process for manufacturing an orally administrable solid medicine comprising a medicinal component or a solid medicinal component having an uncomfortable taste and a polymer film coating covering the medicinal component or the solid medicinal component, wherein the polymer coating comprises, based on the total weight of the orally administrable solid medicine, the following components:

(a) 5-40 wt.% of a film forming polymer, and

(b) 2-60 wt.% of an adhesion/aggregation preventing agent selected from the group consisting of starch particles having a particle size of 15 $\mu$m or smaller and fine crystalline cellulose particles having a particle size of 25 $\mu$m or smaller,

the process comprising the steps of:

2

(1) providing a first dispersion of the film forming polymer in water or a solution of the film forming polymer in an organic solvent and/or water;

(2) dispersing the adhesion/aggregation preventing agent into the first dispersion or the solution to form a second dispersion;

(3) coating the medicinal component or the solid medicinal component with the second dispersion, and

(4) drying the coated medicinal component or solid medicinal component to prepare the orally administrable solid medicine.

Any medicinal components or solid medicinal components can be used in the present invention, so long as such medicinal components or solid medicinal components give uncomfortable tastes such as bitter taste and acid taste when they are orally taken. The medicinal components include macrolide type antibiotics (e.g., erythromycin), $\beta$ -lactam type antibiotics (e.g., penicillin, cephalosporin derivatives), vitamins (e.g., nicotinamide, calcium pantothenate, thiamine nitrate, pyridoxine hydrochloride, riboflavin phosphate), anti-ulcer agents (e.g., cimetidine, 2-N-[3-[3-(1-pyperidinomethyl)phenoxy]propyl]amino-5-amino-1,3,4-thiadiazole), anti-allergy agents (e.g., Tazanolast™), berberine chloride, quinine sulfate, and the like.

The polymer film coating covering the medicinal components or the solid medicinal components comprises a film forming polymer and an adhesion/aggregation preventing agent.

The film forming polymer includes gastric juice soluble polymers such as dimethylaminoethylmethacrylate-methacrylic acid copolymer (Eudragit® E produced by Röhm Pharm GmbH), polyvinylacetal-diethylaminoacetate (AEA produced by Sankyo Co., Ltd.); water insoluble polymers such as ethyl acrylate-methacrylic acid copolymer (Eudragit ® NE produced by Röhm Pharm GmbH), ethyl acrylate-methyl methacrylate-trimethylammoniumethyl methacrylate chloride copolymer (Eudragit ® RL or Eudragit ® RS, produced by Röhm Pharm GmbH) and ethyl cellulose; intestinal juice soluble polymers such as methacrylic acid-ethyl acrylate copolymer (Eudragit® L30D-55 produced by Röhm Pharm GmbH), hydroxypropylmethyl cellulose phthalate (HP-55™ produced by The Shin-Etsu Chemical Co., Ltd.) and hydroxypropylmethyl cellulose acetate succinate (AQOAT™ produced by The Shin-Etsu Chemical Co., Ltd.); or water-soluble polymers such as hydroxypropylmethyl cellulose (TC-5™ produced by The Shin-Etsu Chemical Co., Ltd.), hydroxypropyl cellulose (HPC™ produced by Nippon Soda Co., Ltd.) and polyethylene glycol (PEG6000™). The gastric juice soluble polymers, water-insoluble polymers and intestinal juice soluble polymers may be used alone or in combination with the water-soluble polymers.

The adhesion/aggregation preventing agent usable in the present invention is starch particles having an average particle size of 15 $\mu$m or smaller or fine crystalline cellulose particles having an average particle size of 25 $\mu$m or smaller.

Any starch can be used so long as they are fine particles. The starch includes rice starch (e.g., Micro pearl ® produced by Shimada Chemical Co., Ltd.), corn starch, wheat starch, sweet potato starch, etc. It is preferable that the average particle size is 15 $\mu$m or smaller, in order to provide sufficient adhesion or aggregation preventing property.

Any kind of cystalline cellulose can be used so long as it is in fine powder form (e.g., Avicel® PH-M06, M15 and M2 5 produced by Asahi Chemical Industry Co., Ltd.). It is preferable that fine crystalline cellulose have an average particle size of 25 $\mu$m or smaller, in order to provide sufficient adhesion or aggregation preventing property.

These agents may be used alone or in combination thereof.

The film forming polymer is used in an amount of 5-40wt. %, preferably 10-20 wt.%, based on the weight of the resultant medicine. When the amount is less than 5 wt.%, a film cannot be formed. When the amount is higher than 40 wt.%, the elution of the medicinal component is prevented, and therefore such an amount is not preferable.

When the adhesion/aggregation preventing agent is used in an amount less than 0.4 parts by weight per one part by weight of the film forming polymer, it is difficult to prevent the adhesion or aggregation of the medicine particles. When adhesion/aggregation preventing agent is used in an amount more than 3.0 parts by weight per one part by weight of the film forming polymer, the solid content is too large to form a sufficient coating. Therefore, the adhesion/aggregation preventing agent is used in an amount of 2-60 wt.%, preferably 5-30 wt.%, based on the weight of the final medicine.

In this specification, the term "medicinal component" means a medicine which does not include any additives, the term "solid medicinal component" means granules, fine granules, pellets or powder which includes the medicinal component and hereinafter the medicinal component and the solid medicinal component are reffered to as "medicinal particles". In particular, the medicinal component without any additives can be coated with the polymer coating when its average particle size is relatively large (e.g., 100 $\mu$m or larger).

EP 0 570 606 A1

The medicinal particles are usually prepared by extrusion granulation, high speed agitation granulation or tumbling fluidized bed granulation. The medicinal particles are then coated with a coating dispersion comprising an adhesion/aggregation preventing agent and a film forming polymer.

The coating dispersion (second dispersion) is usually prepared by dispersing a film forming polymer in water (to form a first dispersion) and/or dissolving the film forming polymer in a suitable organic solvent such as ethanol (to prepare solution); and then dispersing an adhesion/aggregation preventing agent into the first dispersion or solution.

The medicinal particles are coated with the second dispersion. The coating may be carried out, using a conventional apparatus such as a fluidized bed coating machine or tumbling fluidized bed coating machine, without occurring any adhesion and/or aggregation of the medicinal particles. The coated medicinal particles are then dried, to prepare the orally administrable solid medicine.

The medicinal component may be contained in the medicinal particles in an amount of up to 93 wt.% based on the total amount of the coated medicine.

The orally administrable solid medicine of the present invention may be administered as granules, powder or pellets. In case where the medicine is administered to, in particular infants, a sweet component may be blended with the coated medicinal particles, as a dry syrup.

According to the present invention, it is possible to produce, in a high yield, an orally administrable solid medicine wherein uncomfortable taste of a medicinal component is masked and wherein a high bio-availability is obtained, with a conventional coating technique and without using special apparatuses, operations and techniques.

EXAMPLES

The present invention will be further illustrated in detail by reference to the following non-limitative examples and tests. Unless otherwise indicated, "part" and "%" mean those by weight.

The examples will be explained in the order of: (1) the conventional preparation of medicinal particles such as granules, powder and pellets containing a medicinal component having an uncomfortable taste (granulation); (2) film coating of the medicinal particles with a masking coating material comprising a film forming polymer and an adhesion/aggregation preventing agent, according to a conventional coating operation using a conventional film coating machine such as fluidized bed coating machine or tumbling fluidized bed coating machine; and (3) drying the coated medicine particles.

Example 1

(Preparation of pellets containing a medicinal component having a bitter taste)

Formulation

|  | % | g |
|---|---|---|
| Tazanolast™ | 28.6 | 400 |
| Anhydrous dibasic calcium phosphate | 28.3 | 396 |
| Partly pregelatinized starch | 37.1 | 520 |
| Low substituted hydroxypropyl cellulose | 4 | 56 |
| Hydroxypropyl cellulose | 2 | 28 |
| Total | 100 | 1400 |

Granulation

The components listed in the formulation except for hydroxypropyl cellulose were introduced into a high speed agitation granulator (Hi-speed Mixer FS-GS-5JD™ manufactured by Fukae Industry Co. Ltd.) and mixed for 2 min. With stirring, the mixed components were granulated with a solution of hydroxypropyl cellulose dissolved with 400ml of ethanol. The resultant fine granules were dried at 50 °C for 5 hr. in a ventilation dryer and then passed through a vibration screen of 32 mesh.

4

Coating Operation of Masking Film

(1) Composition of Coating Liquid

|  | part | g |
|---|---|---|
| 30% water dispersion of ethyl acrylate-methacrylic acid (Eudragit® NE30D) | 24.5 | 98 (Solid Content: 29.4g) |
| Hydroxypropylmethyl cellulose (TC-5R™) | 0.15 | 0.6 |
| Rice starch (Micro Pearl® : average particle diameter: 4.9 $\mu$m ) | 15 | 60 |
| Purified water | 60.35 | 241.4 |
| Total | 100 | 400 |

(2)Coating Operation

210g of the fine granules was introduced into a tumbling fluidized bed coating machine with agitation blades (New/marumerizer NQ-Labo™ manufactured by Fuji Paudal Co., Ltd.) and was coated with 400g of the coating liquid under the conditions that the inlet air temperature was 35 °C ;the air pressure was 50mmH$_2$O; the rate of air introduced was 0.5m$^3$/min.; a rotation speed was 500rpm;the spray pressure was 1kg/cm$^2$; and the amount of a coating liquid introduced was 5cm$^3$/min. The coating operation was continued for about 80 min. Then, the fine granules were dried overnight at 40°C , to yield 288g of fine granules containing about 20% of Tazanolast™, wherein its bitter taste was masked.

Example 2

(Preparation of pellets containing a medicinal component having a bitter taste)

Formulation

|  | % | g |
|---|---|---|
| Tazanolast™ | 20 | 500 |
| Mannitol | 58 | 1450 |
| Partly pregelatinized starch | 20 | 500 |
| Hydroxypropyl cellulose | 2 | 50 |
| Total | 100 | 2500 |

Granulation

The components listed in the formulation except for hydroxypropyl cellulose were introduced into a high speed agitation granulator (Hi-speed Mixer FS-GS-5JD™ manufactured by Fukae Industry Co. Ltd.) and mixed for 2 min. With stirring, the mixed components were granulated with a solution of hydroxypropyl cellulose dissolved with 400ml of ethanol. Then, the same procedure was repeated as Example 1, to yield the fine granules of the present invention.

Coating Operation of Masking Film

(1) Composition of Coating Liquid

|  | part | g |
|---|---|---|
| Dimethylaminoethyl methacrylate-methacrylic acid (Eudragit® E100) | 4 | 200 |
| Rice starch (Micro pear® : average particle size: 4.9 μm) | 16 | 800 |
| Ethanol | 80 | 3800 |
| Total | 100 | 4800 |

(2)Coating Operation

2000g of the fine granules was introduced into a tumbling fluidized bed coating machine with agitation blades (New/marumerizer NQ-230 manufactured by Fuji Paudal Co., Ltd.) and was coated with 4800g of the coating liquid under the conditions that the inlet air temperature was 40 °C ; the air pressure was 50mmH$_2$O; the rate of air introduced was 2.5m$^3$/min.; the rotation speed was 250rpm; spray air amount was 50Nl/min; and the amount of a coating liquid introduced was 40cm$^3$/min. The coating operation was continued for about 2 hr. Then, the fine granules were dried overnight at 40 °C , to yield 2650g of fine granules containing about 13.3% of Tazanolast™ , wherein its bitter taste was masked.

Example 3

(Preparation of pellets containing a medicinal component having a bitter taste)

Formulation

|  | % | g |
|---|---|---|
| 2-N-[3-[3-(1-piperidinomethyl) phenoxy]propyl]amino-5-amino-1,3,4-thiadiazole (WP-834)™ ) | 28 | 420 |
| Anhydrous dibasic calcium phosphate | 38 | 570 |
| Partly pregelatinized starch | 32 | 480 |
| Hydroxypropyl cellulose | 2 | 30 |
| Total | 100 | 1500 |

Granulation

The components listed in the formulation except for hydroxypropyl cellulose were introduced into a high speed agitation granulator (Hi-speed Mixer FS-GS-5JD™ manufactured by Fukae Industry Co. Ltd.) for 2 min. With stirring, the mixed components were granulated with a solution of hydroxypropyl cellulose dissolved with 300ml of ethanol. Then, the same procedure was repeated as Example 1, to yield the fine granules of the present invention.

Coating Operation of Masking Film

(1) Composition of Coating Liquid

|  | part | g |
|---|---|---|
| 30% water dispersion of ethyl acrylate-methacrylic acid (Eudragit® NE30D) | 38 | 190 (Solid content: 57g) |
| Hydroxypropyl cellulose (TC-5R™) | 0.2 | 1 |
| Rice starch (Micro pearl® :average particle size: 4.9 μm) | 13.4 | 67 |
| Purified water | 48.4 | 242 |
| Total | 100 | 500 |
|  |  | (Solid content:125g) |

(2)Coating Operation

375g of the fine glanules was introduced into a fluidized bed coating machine (Uni-Glatt™ manufactured by Ohgawara Seisakusho Co., Ltd.) and spray coated with 500g of the coating liquid under the conditions that the inlet air temperature was 40 °C ; the outlet air temperature was 28°C; the spray pressure was 1kg/cm$^2$; and the amount of a coating liquid introduced was 10cm$^3$/min. The coating operation was continued for about 1 hr. Then, the fine granules were dried overnight at 40°C , to yield 485g of fine granules containing about 20% of WP-834, wherein its bitter taste was masked.

Example 4

(Preparation of granules containing a medicinal component having a bitter taste)

Formulation

|  | % | g |
|---|---|---|
| Nicotinamide | 40 | 200 |
| Lactose | 46 | 230 |
| Corn starch | 12 | 60 |
| Hydroxypropyl cellulose | 2 | 10 |
| Total | 100 | 500 |

Granulation

The components listed in the formulation except for hydroxypropyl cellulose were introduced into a high speed agitation granulator (New Gramachine™ manufactured by Daiwa Kako Kikai Industry Co., Ltd.) and mixed for 2 min. Then, a solution of hydroxypropyl cellulose dissolved with 150ml of ethanol was added to the mixed components with stirring. The wet mixture was extruded in the form of granules using an extrusion granulator provided with a screen of 0.8 mm (Kikusui Seisakusho Co., Ltd.). The granules were introduced into a ventilation dryer, dried at 50°C for 5 hr. and passed through a 18 mesh screen for masking.

Coating Operation of Masking Film

7

(1) Composition of Coating Liquid

|  | part | g |
|---|---|---|
| Dimethylaminoethyl methacrylate-methacrylic acid (Eudragit® E100) | 5 | 30 |
| Fine crystalline cellulose (Avicell® M06: average particle size: 6 $\mu$m) | 3 | 18 |
| Ethanol | 92 | 552 |
| Total | 100 | 600 |

(2)Coating Operation

200g of the granules was introduced into a tumbling fluidized bed coating machine with agitation blades (New/marumerizer NQ-Labo™ manufactured by Fuji Paudal Co., Ltd.) and was coated with 600g of the coating liquid under the conditions that the inlet air temperature was 40 °C ; the air pressure was 50mmH$_2$O; the rate of air introduced was 0.5m$^3$/min.; the rotation speed was 500rpm; the spray pressure was 1kg/cm$^2$; and the amount of a coating liquid introduced was 5cm$^3$/min. The coating operation was continued for about 2 hr. Then, the granules were dried overnight at 40 °C , to yield 240g of granules containing about 16% of nicotinamide, wherein its bitter taste was masked.

Example 5

(Preparation of granules containing a medicinal component having a bitter taste)

Formulation

|  | % | g |
|---|---|---|
| Berberine™ chloride | 20 | 200 |
| Lactose | 50 | 500 |
| Partly pregelatinized starch | 26 | 260 |
| Polyvinyl pyrrolidone K-25 | 4 | 40 |
| Total | 100 | 1000 |

Granulation

The components listed in the formulation except for polyvinyl pyrrolidone were introduced into a high speed agitation granulator (Hi-speed Mixer FS-GS-5JD™ manufactured by Fukae Industry Co., Ltd.) and mixed for 1 min. Into this machine, a solution of polyvinyl pyrrolidone in 250ml of ethanol was dropped. Then, the same procedure was repeated as Example 1, to yield the granules of the present invention.

Coating Operation of Masking Film

(1) Composition of Coating Liquid

| | part | g |
|---|---|---|
| Methyl methacrylate-trimethyl-ammoniummethyl methacrylate chloride (Eudragit® RL) | 5 | 30 |
| PEG 6000 | 0.7 | 4.2 |
| Corn starch | 7.5 | 45 |
| Ethanol | 86.8 | 520.8 |
| Total | 100 | 600.0 |

(2)Coating Operation

200g of the granules was introduced into a tumbling fluidized bed coating machine with agitation blades (New/marumerizer NQ-Labo™ manufactured by Fuji Paudal Co., Ltd.) and was coated with 600g of the coating liquid under the same conditions as Example 4. The coating operation was continued for about 2 hr. Then, the granules were dried overnight at 40 °C, to yield 263g of granules containing about 14.3% of Berberine™ chloride, wherein its bitter taste was masked.

Example 6

(Preparation of pellets containing a medicinal component having a bitter taste)

Formulation

| | g |
|---|---|
| Ibuprofen™ | 100 |
| Lactose | 140 |
| Corn starch | 52 |
| Hydroxypropyl cellulose | 8 |
| Total | 300 |

Granulation

The components listed in the formulation except for hydroxypropyl cellulose were introduced into a tumbling fluidized bed coating machine with agitation blades (New/marumerizer NQ-Labo™ manufactured by Fuji Paudal Co., Ltd.) and mixed for 5 min. Into this apparatus, a solution of hydroxypropyl cellulose dissolved with 150ml of ethanol was sprayed to the mixed components with fluidizing. The wet granules were dried at 50°C for 30 min., taken out from the apparatus, and passed through a 32 mesh screen for masking (288g).

Coating Operation of Masking Film

(1) Composition of Coating Liquid

|  | part | g |
|---|---|---|
| 30% water dispersion of ethyl acrylate-methacrylic acid copolymer (Eudragit® NE30D) | 12 | 75 (Solid content: 22.5g) |
| Rice starch (Micro pearl® : average particle size: 4.9 μm) | 6 | 37.5 |
| Purified water | 82 | 512.5 |
| Total | 100 | 625 |
|  |  | (Solid content: 60g) |

(2)Coating Operation

In the same manner as Example 1 using 150g of the fine granules and 625g of the coating liquid, yielded was 214g of the fine granules of the present invention wherein Ibuprofen™ was contained in an amount of about 23.8% and its bitter taste was masked.

Example 7

(Preparation of dry syrup)

210g of the masked pellets of Example 6 was mixed with 290g of pellets having the following formulation, to produce infantile dry syrup containing 10% of Ibuprofen™.

Formulation

|  | g |
|---|---|
| Succrose powder | 330 |
| Low substituted hydroxypropyl cellulose | 18 |
| Citric acid | 2.4 |
| Sodium citrate | 2.4 |
| Hydroxypropyl cellulose | 7.2 |
| Total | 360.0 |

Granulation

The components listed in the formulation except for hydroxypropyl cellulose were introduced into a tumbling fluidized bed coating machine (New/marumerizer NQ-Labo™ manufactured by Fuji Paudal Co., Ltd.) and mixed for 5 min. Then, a solution of hydroxypropyl cellulose dissolved with 144ml of ethanol was sprayed onto the mixed components with fluidizing. The wet granules were dried at 50 °C for 30 min., taken out from the apparatus, and passed through a 32 mesh screen for masking (339g).

Example 8

(Masking coating of medicinal component having a strong acid taste)

Formulation of Masking Coating Liquid

|  | part | g |
|---|---|---|
| Hydroxypropylmethyl cellulose phthalate (HP-55® manufactured by Shin-etsu Polymer) | 4 | 10 |
| Rice starch (Micro pearl® : average particle size: 4.9 $\mu$m) | 4 | 10 |
| Ethanol | 70 | 175 |
| Purified water | 22 | 55 |
| Total | 100 | 250 |

Coating Operation

200g of ascorbic acid powder (average particle size: 265 $\mu$m) was introduced into a fluidized bed coating machine (Uni-Glatt™ manufactured by Ohgawara Seisakusho Co., Ltd.) and was coated with 250g of the masking coating liquid under the conditions that the inlet air temperature was 40 °C ;the outlet air temperature was 30°C ;the spray pressure was 1kg/cm²; and the amount of the coating liquid introduced was 4ml/min. The coating operation was continued for about 70min. Then, the granules were dried overnight at 40°C to yield 215g of granules containing about 90% of ascorbic acid and wherein the acid taste was masked.

Comparative Example 1

Example 1 was repeated except that talc (average particle diameter: 2.8 $\mu$m) was used in place of rice starch (adhesion/aggregation preventing agent) using the composition of the coating liquid used in the coating operation. During the operation, the adhesion of the medicinal particles to the wall of the coating apparatus was caused. The clogging of the spray gun was also caused. Further, the medicine particles caused caking. Therefore, it had to stop the coating operation, and then to sweep the wall, wash the spray gun and cause the cake to be broken into particles. As a result, it took about 3 hours and a half to complete the whole operation and the yield was only 53.1%.

Comparative Example 2

Example 4 was repeated except that talc (average particle diameter: 2.8 $\mu$m) was used in place of fine crystalline cellulose (Avicel® M06) (adhesion/aggregation preventing agent) using in the composition of the coating liquid used in the coating operation. During the operation, the adhesion of the medicinal particles to the wall of the coating apparatus was caused because static electricity was charged. It took about 3 hours to complete the whole procedure.

(Comparison of Medicines of Examples and Comparative Examples)

Table 1 shows the particle size distribution, average size, yield, and $T_{5\%}$ and $T_{80\%}$ (Dissolution Test according to Paddle method listed in Japanese Pharmacopoeia general test methods under the conditions that the test solution is 900 ml water; rotation is 100 rpm; and temperature is 37°C), and the length of time from when each of the fine granules medicine of Ex.1 and Comp. Ex. 1 is actually received in the mouth until the bitter taste of the medicinal component is noticed. $T_{5\%}$ and $T_{80\%}$ respectively mean the length of time necessary for dissolving 5% or 80% of the medicinal component.

From Table 1, it is clear that in Example 1 the masked fine granules can be obtained at a yield as high as 99.3%, while in Comparative Example 4 using talc, the yield is 53.1%, which is very low as compared with Example 1. This clearly shows that rice starch is superior to talc in its adhesion/aggregation preventing effect.

It took only 3 sec. until the bitter taste of the medicinal component itself (without coating) was noticed from the time when the medicine was taken into the mouth. On the other hand, the fine granules of Example 1 were able to mask the taste for more than 2 min. This length of time is sufficient for the masked medicine.

Table 2 shows the particle size distribution, average size, yield, and $T_{5\%}$ and $T_{80\%}$ (dissolution Test according to Paddle method listed in Japanese Pharmacopoeia general test methods under the conditions that the test solution is 900 ml water; rotation is 100 rpm; and temperature is 37°C), and the length of time until the bitter taste the medicinal component is noticed when the medicine is taken orally, for each of the granule medicine of Example 4 and Comparative Example 2.

In Example 4, the yield of the masked granules was 92.8%, which is very high. On the other hand, in Comparative Example 2, many granules wherein two to several granules were adhered were found. For that reason, the yield was 76.6%, which is lower than the yield of Example 4. In view of these results, it is clear that fine crystalline cellulose (Avicel ® M06) is very effective as an adhesion/aggregation preventing agent, like rice starch in Example 1. The coating material used in Example 4 is a gastric juice soluble polymer. Accordingly, $T_{5\%}$ was 4-5 min. when water was used. However, when J.P. Disintergation Test First Fluid (produced by adding to 2.0g of NaCl, 24.0ml of diluted HCl and the prescribed amount of water to form 1000ml of the final fluid) was used (pH: 1.2), $T_{5\%}$ was about 1 min. and $T_{80\%}$ was 3.5 min. This means that nicotinamide was dissolved very fast. Therefore, the present medicine is effective for preventing the bitter taste and is expected to dissolve rapidly in a stomach after oral administration.

In case of the uncoated granule medicine, the bitter taste was felt as soon as it was received in the mouth. However in Example 4, the bitter taste was masked for more than 4 min.

EP 0 570 606 A1

## Table 1

| Size distribution (%) | Example 1 | | Comparative Example 1 |
|---|---|---|---|
| | Before coating | After coating | After coating |
| 18-32 mesh | ---- | 0.7 | 46.9 |
| 32-48 mesh | 22.8 | 80.9 | 47.0 |
| 48-80 mesh | 54.8 | 17.6 | 3.6 |
| 80-150 mesh | 20.1 | 0.8 | 2.5 |
| 150 mesh pass | 4.4 | ---- | ---- |
| Average size ($\mu$m) | 235 | 368 | 495 |
| Fine granules Yield (%) (32-150 mesh) | ---- | 99.3 | 53.1 |
| Dissolution test | | | |
| $T_{5\%}$ (min.) | ---- | 2.1 | 2.8 |
| $T_{80\%}$ (min.) | ---- | 19.2 | 47.8 |
| Length of time* until feeling bitter taste | about 3 sec. | 2 min.15 sec. | 2 min. 50 sec. |

(Note (*): This time means a length of time until bitter taste is felt from the time when 500 mg of the pellet medicine is taken orally together with 10 ml of water.)

13

## Table 2

| Size distribution (%) | Example 4 | | Comparative Example 2 |
|---|---|---|---|
| | Before coating | After coating | After coating |
| 12-16 mesh | ---- | ---- | 8.7 |
| 16-18 mesh | ---- | 7.2 | 14.7 |
| 18-32 mesh | 100 | 92.8 | 76.6 |
| 32 mesh pass | ---- | ---- | ---- |
| Granule Yield (%) (18-32 mesh) | 100 | 92.8 | 76.6 |
| Dissolution test | | | |
| $T_{5\%}$ (min.) (water) | 0.1 | 4.8 | 4.5 |
| $T_{5\%}$ (min.) | 0.1 | 1.2 | 1.2 |
| $T_{80\%}$ (min.) (First fluid**) | 2.0 | 3.5 | 3.5 |
| Length of time* until feeling bitter taste | about 1 sec. | 4 min. 45 sec. | 4 min. 23 sec. |

(Note (*): This time means a length of time until bitter taste is felt from the time when 625 mg of the granule medicine is taken orally together with 10 ml of water.)
(**) This first fluid means J.P. Disintergation Test First Fluid.

Example 9

Example 1 was repeated by using various starch particles as an adhesion/aggregation preventing agent in order to determine the relation between the particle diameter of the starch particles and the adhesion and/or aggregation preventing effects. The results are shown in below.

14

Table 3

| Starch | Average diameter ($\mu$m) | Apparent specific gravity | adhesion/aggregation preventing effect |
|---|---|---|---|
| Rice | about 5 | 0.31 | very good |
| Corn | about 15 | 0.40 | good |
| Wheat | about 14 | 0.41 | fairly good |
| Potato | about 38 | 0.49 | poor |

As shown in Table 3, good adhesion/aggregation preventing effects can be obtained with starch particles having a particle diameter of 15 $\mu$m or smaller.

Example 10

Example 4 was repeated using various fine cellulose particles as an adhesion/aggregation preventing agent in order to determine the relation between the particle diameter of the cellulose particles and the adhesion and/or aggregation preventing effects. The results are shown in below.

Table 4

| Starch | Average size ($\mu$m) | Apparent specific gravity | adhesion/aggregation preventing effect |
|---|---|---|---|
| Avicel® PH-M06 | 6 | 0.50 | good |
| Avicel® PH-M15 | 15 | 0.55 | good |
| Avicel® PH-M25 | 25 | 0.55 | good |
| Avicel® PH-101 | 40 | 0.30 | poor |

As shown in Table 4, good adhesion/aggregation preventing effects can be obtained with fine cellulose particles having a particle size of 25 $\mu$m or smaller.

**Claims**

1. An orally administrable solid medicine comprising a medicinal component or a solid medicinal component and a polymer film coating covering said medicinal component or a solid medicinal component, wherein said polymer film coating comprises, based on the total weight of the orally administrable solid medicine, the following components:
   5-40 wt.% of a film forming polymer and
   2-60 wt.% of an adhesion/aggregation preventing agent selected from the group consisting of starch particles having an average particle size of 15$\mu$ m or smaller and fine crystalline cellulose particles having an average particle size of 25 $\mu$m or smaller, and mixtures thereof.

2. The orally administrable solid medicine of claim 1, wherein said starch is selected from the group consisting of rice starch and corn starch.

3. The orally administrable solid medicine of claim 1 or 2, wherein said adhesion/aggregation preventing agent is present in an amount of 5-30 wt.% based on the total weight of the orally administrable solid medicine.

4. The orally administrable solid medicine of claim 1,2 or 3, wherein said film forming polymer is selected from the group consisting of dimethylaminoethylmethacrylate-methacrylic acid copolymer, polyvinylacetal diethylaminoacetate, ethyl acrylate-methacrylic acid copolymer, ethyl acrylate-methyl methacrylate-trimethylammoniumethyl methacrylate chloride copolymer, methacrylic acid-ethyl acrylate copolymer, ethyl cellulose, hydroxypropylmethyl cellulose phthalate, hydroxypropylmethyl cellulose acetate succinate, hydroxypropylmethyl cellulose, hydroxypropyl cellulose and polyethylene glycol, and these film forming polymers may be used individually or in combinations thereof.

5. The orally administrable solid medicine of any one of claims 1 to 4, wherein said film forming polymer is present in an amount of 10-20 wt.% based on the total weight of the orally administrable solid medicine.

6. The orally administrable solid medicine of any one of claims 1 to 5, wherein said medicinal component is a medicine having uncomfortable taste such as bitter taste or acid taste.

7. The orally administrable solid medicine of any one of claims 1 to 6, wherein said solid medicinal component is selected from pellets, granules, fine granules or powder which include said medicinal component.

8. A process for preparing an orally administrable solid medicine comprising a medicinal component or a solid medicinal component and a polymer film coating covering said medicinal component or solid medicinal component, wherein said polymer film coating comprises, based on the total weight of the orally administrable solid medicine, the following components:
   5-40 wt.% of a film forming polymer and
   2-60 wt.% of an adhesion/aggregation preventing agent selected from the group consisting of starch particles having an average particle size of 15 $\mu$m or smaller and fine crystalline cellulose particles having an average particle size of 25 $\mu$m or smaller, and mixtures thereof,
   said process comprising the steps of:
   providing a first dispersion of said film forming polymer in water or a solution of said film forming polymer in an organic solvent and/or water;
   dispersing said adhesion/aggregation preventing agent into said first dispersion or said solution to form a second dispersion;
   coating said medicinal component or said solid medicinal component with said second dispersion, and
   drying the coated medicinal component or solid medicinal component to prepare the orally administrable solid medicine.

9. The process of claim 8, wherein said starch is selected from the group consisting of rice starch and corn starch.

10. The process of claim 8 or 9, wherein said adhesion/aggregation preventing agent is used in an amount of 5-30 wt.% based on the total weight of the orally administrable solid medicine.

11. The process of claim 8, 9 or 10, wherein said film forming polymer is selected from a group consisting of dimethylaminoethylmethacrylate-methacrylic acid copolymer, polyvinylacetal diethylaminoacetate, ethyl acrylate-methacrylic acid copolymer, ethyl acrylate-methyl methacrylate-trimethylammoniumethyl methacrylate chloride copolymer, methacrylic acid-ethyl acrylate copolymer, ethyl cellulose, hydroxyopropylmethyl cellulose phthalate, hydroxypropylmethyl cellulose acetate succinate, hydroxypropylmethyl cellulose, hydroxypropyl cellulose and polyethylene glycol, and these film forming polymers may be used individually or in combinations thereof.

12. The process of any one of claims 8 to 11, wherein said film forming polymer is used in an amount of 10-20 wt.% based on the total weight of the orally administrable solid medicine.

**Claims for the following Contracting State : ES**

1. A process for preparing an orally administrable solid medicine comprising a medicinal component or a solid medicinal component and a polymer film coating covering said medicinal component or solid medicinal component, wherein said polymer film coating comprises, based on the total weight of the orally administrable solid medicine, the following components:
   5-40 wt.% of a film forming polymer and
   2-60 wt.% of an adhesion/aggregation preventing agent selected from the group consisting of starch particles having an average particle size of 15 $\mu$m or smaller and fine crystalline cellulose particles having an average particle size of 25 $\mu$m or smaller, and mixtures thereof,
   said process comprising the steps of:
   providing a first dispersion of said film forming polymer in water or a solution of said film forming polymer in an organic solvent and/or water;

dispersing said adhesion/aggregation preventing agent into said first dispersion or said solution to form a second dispersion;

coating said medicinal component or said solid medicinal component with said second dispersion, and

drying the coated medicinal component or solid medicinal component to prepare the orally administrable solid medicine.

2.  The process of claim 1, wherein said starch is selected from the group consisting of rice starch and corn starch.

3.  The process of claim 1 or 2, wherein said adhesion/aggregation preventing agent is used in an amount of 5-30 wt.% based on the total weight of the orally administrable solid medicine.

4.  The process of claim 1, 2 or 3, wherein said film forming polymer is selected from a group consisting of dimethylaminoethylmethacrylate-methacrylic acid copolymer, polyvinylacetal diethylaminoacetate, ethyl acrylate-methacrylic acid copolymer, ethyl acrylate-methyl methacrylate-trimethylammoniumethyl methacrylate chloride copolymer, methacrylic acid-ethyl acrylate copolymer, ethyl cellulose, hydroxypropylmethyl cellulose phthalate, hydroxypropylmethyl cellulose acetate succinate, hydroxypropylmethyl cellulose, hydroxypropyl cellulose and polyethylene glycol, and these film forming polymers may be used individually or in combinations thereof.

5.  The process of any one of claims 1 to 4, wherein said film forming polymer is used in an amount of 10-20 wt.% based on the total weight of the orally administrable solid medicine.

6.  The process of any one of claims 1 to 5, wherein said medicinal component is a medicine having uncomfortable taste such as bitter taste or acid taste.

7.  The process of any one of claims 1 to 6, wherein said solid medicinal component is selected from pellets, granules, fine granules or powder which include said medicinal component.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| E | DATABASE WPIL<br>Week 9233,<br>Derwent Publications Ltd., London, GB;<br>AN 273994<br>& JP-A-4 187 630 (WAKAMOTO PHARM CO LTD) 6 July 1992<br>* abstract *<br>--- | 1-12 | A61K9/50 |
| X | DATABASE WPIL<br>Week 8838,<br>Derwent Publications Ltd., London, GB;<br>AN 266524<br>& JP-A-63 192 725 (SHINETSU CHEM IND KK)<br>10 August 1988<br>* abstract *<br>--- | 1-12 | |
| X | EP-A-0 076 428 (TANABE SEIYAKU CO., LTD.)<br>* page 14, line 20 - page 15, line 4 *<br>* page 18, line 5 *<br>* page 18, line 14 *<br>* page 19, line 1 - line 15 *<br>* page 24 - page 25; examples 2,3 *<br>--- | 1-7 | |
| X | EP-A-0 133 827 (SEPPIC)<br>* the whole document *<br>--- | 1,3-7 | TECHNICAL FIELDS SEARCHED (Int. Cl.5)<br><br>A61K |
| X | WO-A-9 107 949 (NATIONAL RESEARCH DEVELOPMENT CORPORATION)<br>* page 18, line 18 - line 30 *<br>--- | 1-12 | |
| X | EP-A-0 347 748 (ABBOTT LABORATORIES)<br><br>* the whole document *<br>--- | 1,3-8, 11,12 | |
| X | EP-A-0 468 247 (MERZ & CO. GMBH & CO.)<br><br>* page 3, line 30 - line 54 *<br>---<br>-/-- | 1,3-8, 11,12 | |

The present search report has been drawn up for all claims

| Place of search | Date of completion of the search | Examiner |
|---|---|---|
| THE HAGUE | 29 JANUARY 1993 | BENZ K.F. |

EPO FORM 1503 03.82 (P0401)

European Patent
Office

EUROPEAN SEARCH REPORT

Application Number

EP    92 10 8360
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 169 319 (COLOCRON, INC.)<br>* page 1, line 1 - line 21 *<br>* page 8, line 4 - line 5 *<br>* page 16 - page 17; examples 16,17 *<br><br>----- | 1-12 | |
| | | | TECHNICAL FIELDS SEARCHED (Int. Cl.5 ) |

The present search report has been drawn up for all claims

| Place of search | Date of completion of the search | Examiner |
|---|---|---|
| THE HAGUE | 29 JANUARY 1993 | BENZ K.F. |

EPO FORM 1503 03.82 (P0401)